# EUROPEAN PATENT SPECIFICATION

(11) **EP 2 409 666 B1**
(45) Date of publication and mention of the grant of the patent: **16.07.2014**
(21) Application number: 09806040.3
(22) Date of filing: 09.12.2009
(51) Int. Cl.: A61C 8/00, A61B 5/22, A61F 5/56

(54) **DENTAL ABUTMENT WITH A FORCE TRANSDUCER AND AN INTERFACE FOR CONNECTION TO A NERVE**
DENTALES ABUTMENT MIT EINEM KRAFTSENSOR UND EINER SCHNITTSTELLE ZUR VERBINDUNG ZU EINEM NERV
PILIER DENTAIRE AVEC UN CAPTEUR DE FORCE ET UNE INTERFACE POUR CONNECTION À UN NERF

(30) Priority: 16.03.2009 PT 09104438
(43) Date of publication of application: 25.01.2012
(73) Proprietor: Clínica Médica Santo António De Joane, Lda., 4050-319 Porto (PT)
(72) Inventor: SOBRADO MARINHO, Jorge Serafim, P-4050-322 Porto (PT)
(74) Representative: Ferreira, Maria Silvina
(86) International application number: PCT/IB2009/055607
(87) International publication number: WO 2010/106401

(56) References cited:
- WO-A1-94/28828
- JP-A- 2008 272 322
- US-A- 5 078 153
- CLAES W ET AL: "A 136-/spl mu/w/channel autonomous strain-gauge datalogger" IEEE JOURNAL OF SOLID-STATE CIRCUITS, IEEE SERVICE CENTER, PISCATAWAY, NJ, US, vol. 38, no. 12, 1 December 2003 (2003-12-01), pages 2280-2287, XP011104271 ISSN: 0018-9200

## Description

### Technical Field of the Invention

This invention has applications in Medicine, Neurology, Stomatology, Dentistry, Maxillofacial Surgery and Gerontology, allowing the restoration of proprioceptive and tactile sensitivity in chewing, as well as improving balance capabilities and stereotaxy in edentulous individuals or partial edentulous. Using Nanotechnology, Microelectronics, Bionics and Biophysics technologies.

### Background of the Invention

The document DE3444780 advocates the use of a dental implant constituted of three layers of material, namely: 1 - Titanium as its core, 2 - Inter Middle surface coating consisting of bioactive ceramic and 3 - Carbon fibers, fixed to the outer surface of the bioactive ceramic. The inventor says that the sensitivity given by the implant comes from the bone transmission of the loads received by the implant, and distributed to the periphery of the bone by filaments of the carbon fiber.

There are today several procedures for teeth replacement based on dental implants technology, which can be divided into two major groups:
1 - Removable prostheses atop implants are used during the day and taken after meals and before bedtime. The connectors that attach them to the implants have only the purpose of allowing a minimum of stability and security during phonation, respiration, and mastication. The forces unleashed by the work of mastication are not transmitted to the implants by the axis of insertion of those, but by tangential plans and therefore not perceived through bone conduction.
2 - Fixed prostheses atop implants restrained by fixed and permanent devices, such as pillars and screws adapted to the head or base of the implants. These prosthetic devices ensure the solidity of the set (prosthesis /implants), and thus the transmission of the chewing forces directly to the structure of the implants, which will dissipate them to the surrounding bone. The bone sensitivity is a vague visceral sensitivity, inaccurate, not prejudiced much like the deep visceral abdominal sensitivity. Patients whom were placed implant supported fixed prostheses, are struggling with the problem to adequate the intensity of chewing forces to the type of food, its texture and hardness, and sometimes is frequent the phenomenon of overload, which leads to fatigue of the material used in the manufacture of prostheses, whether in metal and ceramic, acrylic, or totally zirconium minerals and ceramics. The appearance of micro cracks after a while of use (which will be as fast as the stiffness lowers the material in question) will lead to fracture of the materials used, and even in some cases, structural breaks such as disruption of screws and abutments that lead to permanent damage of implanted supported prosthesis.

The document DE3444780 aims to give a morphological configuration of the tooth root implant in what concerns mobility, strength, and sensitivity to pressure, but has no specific mechanism that ensures the generation, transport and delivery of an electrical stimulus that excites the nerve endings responsible for tooth sensitivity.

The JP2008272322 document describes an implant for fixing an artificial tooth to an alveolar bone. The implant includes a piezoelectric sensor for generating a feeble electric current when pressurised. This feeble electric current stimulates a nerve existing close to the implant.

The WO9428828 document describes a device that stops or at least diminishes bruxism using a biofeedback system which detects pathological bruxism and stops it. The device comprises at least one pressure sensor connected to a transponder element, which is connected to a pulse generator. This generator transmits an electrical impulse to a conducting splint anchor. The splint is worn on the palate of the mouth and the pressure sensors are extended over the upper jaw teeth.

### Summary

The present invention describes a universal prosthetic dental abutment with a bionic device sensitive to mastication and mandibular occlusion, which bionic device comprises a transducer that transforms the mechanical stress on the jaw into an electrical pulse, a guiding wire for directing said electrical pulse to an interface card suitable for interfacing to nerve endings, in particular of the trigeminal nerve, wherein said interface card has a neuron-compatible membrane in polyglycon or sodium polyglyconate, in which are embedded one or more of a copper conductor, a gold plate, a plate coated with gold and a titanium mesh.

In a preferred embodiment, the universal prosthetic dental abutment further comprising a data processing means adapted to produce an electrical pulse, which is in direct gradual relation to the mechanical stress, and a power source, in particular a battery.

In another preferred embodiment, the transducer used in the universal prosthetic dental abutment is piezoelectric.

Inyet another preferred embodiment, the guiding wire used in the universal prosthetic dental abutment has a copper core, an insulating coating, and a biocompatible coating.

In a preferred embodiment, the insulating coating used in the universal prosthetic dental abutment comprises silicone or polytetrafluoroethylene, and the bio-compatible coating comprises a titanium mesh.

The invention also encloses a dental implant comprising to above mentioned universal prosthetic dental abutment.

The present invention is greatly useful for the areas of Medicine, Neurology, Stomatology, Dentistry, Maxillofacial Surgery and Gerontology, and utilizes high-tech Nanotechnology Electronics and Biomechanics technology.

The invention which it is intended to present has a structural advantage which can be used in any implant brand available in the market, already placed into the bone or to be osseointegrated. It is not necessary to change the production line to produce implants with particular surface features, designed for dental sensitivity. The Pillar with the bionic device that generates the electrical pulse for nerve stimulation may be installed at any time after placement of an implant prior and after the osseointegration is verified.

The invention which it is intended to present has also a functional advantage of generating an electrical pulse of proprioceptive nerve stimulation whose magnitude is directly related to the intensity of the masticatory forces of occlusion. Thus, it becomes possible to provide the brain of information feedback about the magnitude and type of chewing forces that are being developed by the stomatognathic system at that time.

This information provides efferent regulation information to the brain and modulation of muscle contraction forces of the stomatognathic system which would save the whole set of implants/fixed prosthesis on implants to the structural stress forces overload, which are responsible of causing fatigue of the material and for structural microfractures.

The advantages of the invention, over what is already available on the market, also include:
1. Replacement of teeth sensitive - which is an important function for the control of posture and body balance.
2. Reinstatement of chewing sensitivity - which contributes to a better balance and preservation of movement in older patients.
3. Adaptable to any implant already on the market - can be placed even in patients who already have implants in the mouth.
4. Delivery of an electrical stimulation corresponding to the pressure exerted during mastication, directly on the nerve endings of the trigeminal nerve - thus allowing a proprioceptive sensibility far more perceptive than that which is perceived only by bone conduction.
5. Prevention of overload (over-load) - that is exerted by mastication forces on the prosthetic devices or of the anatomical substitutes that are anchored on the implants, in this way can be avoided material fatigue accidents or structural joint failure in implant/prosthesis of and thus decreasing dental office visits for repair of installed dentures.
6. Enhanced understanding of texture and hardness of food - allowing a more efficient chewing with greater satisfaction and pleasure for the patient, increasing the confidence of the patient, and therefore the demand of the techniques of oral rehabilitation with dental implants.

The invention which it is intended to present is based on the concept of using any type of implant therefore isn't necessary to produce implants with specially designed surfaces to provide sensitivity to the chewing and dental occlusion forces.

The sensitivity is transmitted by an electrical impulse generated in a structure that is fixed to the head of the implant, (ahead referred as pillar/abutment) and lodging inside a bionic mechanism that converts a compressive force into an electrical impulse which can be perceived by the nerve endings of the trigeminal nerve.

The invention which it is intended to present also has a structural advantage that can be used in any type of implant independently of its brand, already osseointegrated or to be implanted, because:
1 - There is no need to change the production line to make implants with particular surface features designed for sensitive teeth.
2 - The Pillar with the bionic device that generates the electrical pulse for nerve stimulation can be installed at any time after placement of an implant prior and after verifying the osseointegration.

The invention which it is intended to present has a functional advantage of generating an electrical pulse of nerve proprioceptive stimulation whose magnitude is directly related to the intensity of the masticatory forces of occlusion.

The present invention has a bionic device housed in an implant abutment that makes it sensitive to chewing pressure forces (or other related matters). The forces are converted into electrical impulses of suitable magnitude and intensity to be perceived by the nerve endings of the trigeminal nerve. The electrical impulse is carried by a copper coated conducting wire with biocompatible sheath in which exists a distal platform of wire mesh wrapped in titanium poligluconato sodium and adapts to the mental nerve endings or infra - orbicular nerve , (both are branches of the trigeminal nerve). Thus, it becomes possible to provide the brain feedback information about the magnitude and type of chewing forces that are being developed by the stomatognathic system at that time. This will provide the brain efferent information of regulation and modulation of muscle contraction forces of the stomatognathic system which will save the whole set (implant/ fixed prosthesis implant) from the structural stress forces overload, that cause fatigue of the material and the structural microfractures.

The advantages of the invention, over what is already known, are:
1. Dental sensitivity, which is an important function for control of posture and body balance.
2. The chewing sensitivity had never before been valued by different implant manufacturers, but the sensitivity function of natural teeth, contributes according to current scientific research, for a better balance and preservation of motion in patients over 70 years of age.
3. This invention can be adapted to any implant already on the market and can be placed even in patients who already have implants in the mouth.
4. While the invention DE3444780 is based on the principle of conducting the sensitivity through the bone surrounding the implant, the present invention performs the delivery of an electrical stimulation corresponding to the pressure exerted during mastication, directly on the nerve endings of the trigeminal nerve, allowing a proprioceptive and discriminative sensitivity much more precise than that which is perceived only by bone conduction, in this case it's a deep undefined visceral sensitivity.
5. The economic importance of the invention lies in three aspects:
   a. Prevents the occurrence of the phenomenon of overload (over-load) exerted by the forces of chewing on the prosthetic device or in the anatomical replacement devices of anchored implants. Thus can avoided fatigue accidents or structural damage of the material of the whole implant prosthesis set. This will decrease the patient visits to the dental clinic for repairing the installed prostheses.
   b. By contributing to a better understanding of texture and hardness of food, one will chew more effectively with greater satisfaction and pleasure for the patient. Increasing the confidence of the patient, and therefore the demand of the techniques of oral rehabilitation with dental implants.
   c. By allowing the perception of the relative position of the mandible in relation to the head and body, provides a spatial orientation improved with gains in gait and balance.

### Description of the Figures

For an easier understanding of the invention we are attaching the figures which represent exemplary embodiments of the invention which, however, are not intended to limit the scope of this invention.

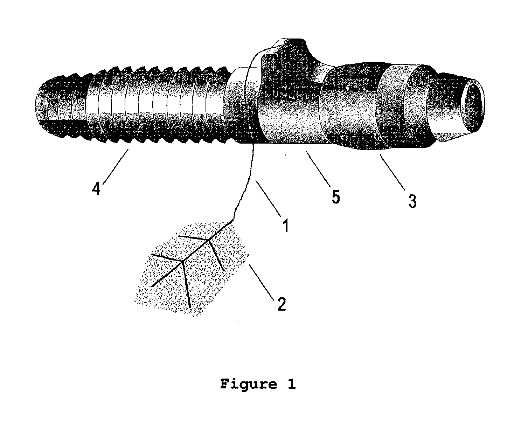

FIG. 1: Schematic representation of the device.

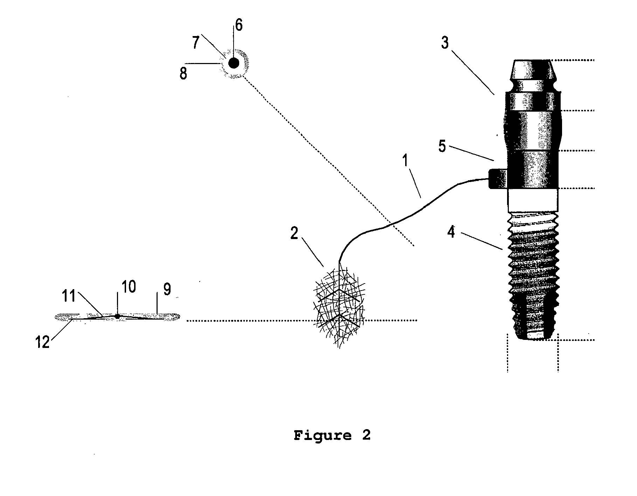

FIG. 2: Detailed schematic representation of the device.

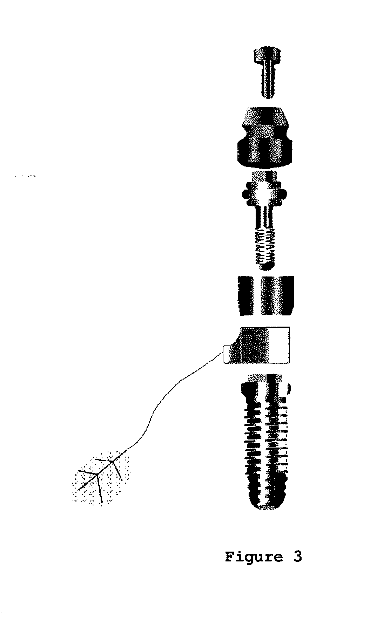

FIG. 3: Schematic representation of the mechanical assembly of the entire device and prosthetic abutment.

### Description of invention

This invention represents a fundamental improvement to the majority of dental implants marketed so far as it nears the anatomical replacement devices (fixed on dental implants) to the primary function of the teeth during chewing, which is to provide information to the brain of the amount of force necessary and sufficient for proper chewing of food intake at a given time. So far all the technology around the implants, has focused on the phenomenon of osseointegration, the demand for new designs of the profile of the implants, together with the search for new types of surface, allowing a better biocompatibility and therefore a more early healing, have proved useful so far as resolving only the aspect of speed of osseointegration, against all the biofunctional of the set (Implant / dental prosthesis). Still remains the problem of possible overload of the chewing forces because the brain has difficulty to evaluate the hardness of the diet, and thus can't send instructions to the muscles of mastication to give an appropriate amount of contractile force to the effort of chewing in real time.

A preferred implementation of the present invention consists of 5 five elements:
1 - Modified prosthetic abutment possessing proper fit for internal or external connection implants.
2 - Micro-chip equipped with a pressure evaluation device, and a data processing device and transduction of mechanical forces into gradual electrical impulses directly related to the intensity scale of masticatory pressure or occlusion.
3 - Unit power supply (battery) to feed the operation of the micro-chip.
4 - Electrical conducting wire generated in the micro-chip, consisting of 3 layers: a) capillary inner wire of copper or other electrically conductive metal. b) First coating the wire, carried out with inert material and electrically non-conducting (Teflon). c) External coating consisting of titanium mesh to be biologically compatible and / or mucus integratable.
5 - Card interface with the nervous structures, consisting of a core of metal plates disposed radially and embedded within the material poliglicano.

Explaining the operation of the invention, shall be conducted as follow:
During the normal process of chewing are applied forces on the modified abutment, which are detected by the micro-chip that turns them into direct contact with its magnitude, into electrical impulses that are generated in the micro-chip and sent by the conductor wire, to the interface card implemented in the neighborhood of the nerve ending, which will deliver the electrical impulses to the nervous tissue. The nerve endings of a nerve of the V cranial pair, leads the stimulus to the centers of the V cranial pair which will be integrated and interpreted corresponding then a motor response to all the muscles of the stomatognathic system.

The most common form of implementation of the invention includes the placement of each modified pillars atop implants located in the four quadrants of the mouth: hemi-maxillary top right hemi-maxilla left, right hemi-mandible, left hemi-mandible. Thus we will have a device to stimulate each of the four endings of nerve-root respectively, V2, V3 and right and left of the fifth cranial nerve, or trigeminal nerve.

The main features of the present invention are:
1. Universal prosthetic abutment, adaptable to any dental implant.
2. Micro-chip residing in the pillar, author of the electrical stimulus in intensity and frequency adapted to the sensory nerve endings of the Fifth Cranial pair.
3. System of transmission of electrical stimulation through an insulated cable and with super thin diameter, ending in a film of noble metal, coated with poliglicano, which will sit directly on the nerve endings of the trigeminal nerve.

Preferable features of the present invention are:
1. The transport charges of electrical stimulation from the outset the abutment on the implant until the termination of the roots V2 or V3 of the trigeminal nerve.
2. The chip that converts the forces of pressure on electrical stimulation with amplitude and frequency modulated to be perceived by the nerve endings.
3. The possibility of placing the chip on pillars "new ad" implant already installed in the oral cavity.

### Table of Reference Signs

(1) represents the guiding wire of the electrical impulses,
(2) represents the interface board with the nervous structures,
(3) represents the prosthetic pillar,
(4) represents the prosthetic pillar,
(5) represents the processing device and force transducer,
(6) represents the core of the guiding wire, namely in copper,
(7) represents the insulating coating of the guiding wire, namely in silicone,
(8) represents a biocompatible coating the guiding wire, namely in titanium mesh,
(9) represents a neuron-compatible membrane, namely in poliglicano,
(10) represents a bio-compatible mesh, namely in titanium
(11) represents a conductor/driver of the interface card, namely in copper,
(12) represents a plate, namely in gold or gold-plated.

## Claims

1. A universal prosthetic dental abutment with a bionic device sensitive to mastication and mandibular occlusion, which bionic device comprises a transducer (5) that transforms the mechanical stress on the jaw into an electrical pulse, a guiding wire for directing said electrical pulse to an interface card (2) suitable for interfacing to nerve endings, in particular of the trigeminal nerve, wherein said interface card (2) has a neuron-compatible membrane in polyglycon or sodium polyglyconate, in which are embedded one or more of a copper conductor, a gold plate, a plate coated with gold and a titanium mesh.

2. The universal prosthetic dental abutment of claim 1 further comprising a data processing means (5) adapted to produce an electrical pulse, which is in direct gradual relation to the mechanical stress, and a power source, in particular a battery.

3. The universal prosthetic dental abutment of claim 1 or 2, wherein the transducer is piezoelectric.

4. The universal prosthetic dental abutment of any of the preceding claims, wherein the guiding wire has a copper core (6), an insulating coating (7), and a biocompatible coating (8).

5. The universal prosthetic dental abutment of claim 4, wherein the insulating coating (7) comprises silicone or polytetrafluoroethylene, and the bio-compatible coating (8) comprises a titanium mesh.

6. A dental implant comprising the universal prosthetic dental abutment of any of the preceding claims.

## Patentansprüche

1. Ein universeller prosthetischer Zahnpfosten mit einer kau- und kieferverschlussempfindlichen bionischen Vorrichtung, welche bionische Vorrichtung einen Wandler (5), der die mechanische Spannung auf der Backe in einen elektrischen Impuls umwandelt, einen Führungsdraht zum Leiten des elektrischen Impulses an eine Schnittstellenkarte (2) geeignet für die Anbindung an Nervenenden, insbesondere des Trigeminus, umfasst, wobei die genannte Schnittstellenkarte (2) eine neuronkompatible Membran aus Polyglycon oder Natrium Polyglyconat hat, in der ein oder mehrere aus einem Kupferleiter, einer Goldplatte, einer Gold beschichteten Platte und einem Titan-Mesh eingebettet sind.

2. Der universelle prosthetische Zahnpfosten nach Anspruch 1, weiter umfassend ein Datenverarbeitungsmittel (5), das angepasst ist, um einen elektrischen Impuls zu erzeugen, der in direktem schrittweisen Zusammenhang mit der mechanischen Spannung und einer Energiequelle, insbesondere einer Batterie, steht.

3. Der universelle prosthetische Zahnpfosten nach Anspruch 1 oder 2, wobei der Wandler piezoelektrisch ist.

4. Der universelle prosthetische Zahnpfosten nach einem der vorhergehenden Ansprüche, wobei der Führungsdraht einen Kupferkern (6), eine isolierende Beschichtung (7) und eine biokompatible Beschichtung (8) aufweist.

5. Der universelle prosthetische Zahnpfosten nach Anspruch 4, wobei die isolierende Beschichtung (7) Silikon oder Polytetrafluorethylen und die biokompatible Beschichtung (8) einen Titan-mesh umfasst.

6. Ein Zahnimplantat umfassend den universellen prosthetischen 2ahnpfosten nach einem der vorhergehenden Ansprüche.

## Revendications

1. Un support dental prothétique universel, avec un dispositif bionique qui est sensible à la mastication et à l'occlusion mandibulaire, le susdit dispositif bionique comprenant un capteur (5) qui transforme la pression mécanique sur le maxillaire en une impulsion électrique, un guide-fil pour guider la susdite impulsion électrique à une carte d'interface (2) convenable pour l'accouplement aux terminaisons nerveuses, en particulier au nerf trijumeau, la susdite carte d'interface (2) possédant une membrane compatible avec un neurone, en polyglycon ou en polyglyconat de sodium, dans laquelle un ou plusieurs conducteur(s) à cuivre, une plaque en or, une plaque avec un revêtement en or et un filet en titane sont emboutis.

2. Le support dental prothétique universel, selon la revendication 1, comprenant en outre un moyen de traitement des données (5) qui est adapté pour générer une impulsion électrique qui est en relation graduele directe avec la pression mécanique, et une source d'alimentation, en particulier une batterie.

3. Le support dental prothétique universel, selon la revendication 1 ou 2, dans lequel le capteur est piézoélectrique.

4. Le support dental prothétique universel, selon une quelconque des revendications précédentes, dans lequel le guide-fil a un noyau (6) en cuivre, un revêtement (7) isolant, et un revêtement (8) biocompatible.

5. Le support dental prothétique universel, selon la revendication 4, dans lequel le revêtement (7) isolant comprend du silicone ou du polytétrafluoréthylène, et le revêtement (8) biocompatible comprend un filet en titane.

6. Un implant dentaire, comprenant le support dental prothétique universel selon une quelconque des revendications précédentes.
